# EUROPEAN PATENT APPLICATION

(11) **EP 3 517 177 A1**
(43) Date of publication of application: **31.07.2019**
(21) Application number: 18153949.5
(22) Date of filing: 29.01.2018
(51) Int. Cl.: A61Q 5/00, A61Q 19/00, A61K 8/81, C08L 33/10, C08L 33/14

(54) **COMPOSITION FOR INCREASING THE VISCOSITY OF AN OIL**

(71) Applicant: BASF SE, 67056 Ludwigshafen am Rhein (DE)
(72) Inventor: Millard, Pierre-Eric, 67056 Ludwigshafen (DE); Zimmermann, Tobias Joachim, 67056 Ludwigshafen (DE); Langlotz, Bjoem, 67056 Ludwigshafen (DE)
(74) Representative: BASF IP Association

(57) **Abstract**

The present invention relates to a composition comprising a first polymer and a second polymer, wherein the first polymer comprises repeating units derived from a monomer A which is selected from the group consisting of an alkyl ester of acrylic acid having at least 8 C-atoms, an alkyl ester of methacrylic acid having at least 9 C-atoms, and mixtures thereof, a monomer B which is an ethylenically unsaturated, polymerizable monomer having an acid group, and optionally at least one monomer C, wherein each monomer C is an ethylenically unsaturated, polymerizable monomer different from monomer A and different from monomer B, wherein the second polymer comprises repeating units derived from a monomer D which is selected from the group consisting of an alkyl ester of acrylic acid having at least 8 C-atoms, an alkyl ester of methacrylic acid having at least 9 C-atoms, and mixtures thereof, a monomer E which is an ethylenically unsaturated, polymerizable monomer having a basic group, and optionally at least one monomer F, wherein each monomer F is an ethylenically unsaturated, polymerizable monomer different from monomer D and different from monomer E. Furthermore, the present invention relates to a kit of parts comprising the said two polymers. Furthermore, the present invention relates to the use of the said composition or of the said kit of parts to increase the viscosity of an oil.

## Description

The present invention relates to a composition comprising a first polymer and a second polymer, wherein the first polymer comprises repeating units derived from a monomer A which is selected from the group consisting of an alkyl ester of acrylic acid having at least 8 C-atoms, an alkyl ester of methacrylic acid having at least 9 C-atoms, and mixtures thereof, a monomer B which is an ethylenically unsaturated, polymerizable monomer having an acid group, and optionally at least one monomer C, wherein each monomer C is an ethylenically unsaturated, polymerizable monomer different from monomer A and different from monomer B, wherein the second polymer comprises repeating units derived from a monomer D which is selected from the group consisting of an alkyl ester of acrylic acid having at least 8 C-atoms, an alkyl ester of methacrylic acid having at least 9 C-atoms, and mixtures thereof, a monomer E which is an ethylenically unsaturated, polymerizable monomer having a basic group, and optionally at least one monomer F, wherein each monomer F is an ethylenically unsaturated, polymerizable monomer different from monomer D and different from monomer E. Furthermore, the present invention relates to a kit of parts comprising the said two polymers. Furthermore, the present invention relates to the use of the said composition or of the said kit of parts to increase the viscosity of an oil.

Only few viscosifier technologies (i. e. technologies or substances for increasing the viscosity, also called "thickener") for hydrophobic solvents (hereinafter, hydrophobic solvent and oil are used synonymously) are commercially available. Fumed silica generates a rather high viscosity only when a high concentration is used and the dispersion generated is highly turbid. Known polymeric materials, e.g. commercially available polyamide derivatives (e.g. OleoCraft® polymers obtainable from Croda) are hard to handle because the solid materials need a high temperature to be dissolved in a medium. Moreover, the dose needed to be effective is high, the final viscous solution is hard to pump because the shear thinning behavior is poor.

Semenycheva et al, Russian Journal of Applied Chemistry, 2014, vol. 87, no. 2, pp. 224-229 reports on copolymers of alkyl methacrylates with vinyl butyl ether and their use for thickening petroleoum oil.

US 2006/0185850 discloses a viscosified treatment fluid comprising water, a carbonyl-containing first compound and an amine-containing second compound.

US 4,486,316 discloses an aqueous composition containing an effective amount of an additive consisting of an emulsion polymerized interpolymer of an olefinically unsaturated carboxylic acid monomer selected from the group consisting of acrylic acid, methacrylic acid and mixtures thereof and at least one other non-carboxylated polymerizable monomer selected from the class consisting of methyl acrylate, ethyl acrylate, methyl methacrylate and mixtures thereof, said interpolymer having a molecular weight of from about 1,000,000 to about 4,000,000, the API apparent viscosity of an aqueous dispersion of said interpolymer containing 0.5 lbs. of polymer solids per barrel of said dispersion in a pH range above about 6 being at least 600% greater than the API apparent viscosity of said dispersion in a pH range below about 6, the pH of said aqueous composition being above about 7, said additive serving to viscosify a drilling fluid.

US 2011/0214864 discloses an invert emulsion drilling fluid having an oleaginous continuous phase, a non-oleaginous discontinuous phase, and a hydrophobic amine additive, wherein the drilling fluid with the hydrophobic amine additive, when compared to the drilling fluid without the hydrophobic amine additive, restricts the increase in plastic viscosity to 60% or less and has a characteristic selected from the group consisting of: an increased yield point; an increased low shear yield point; an increased gel strength; and any combination thereof.

WO 2015/011412 discloses a latex of filamentous polymer particles, said particles being crosslinked, block copolymers synthesized by controlled radical emulsion polymerization, in the form of cylinders having a length/diameter ratio of greater than 100 and added to said solution at a content by weight of a minimum of 100 ppm. Ionic monomers are disclosed.

US 7 829 071 discloses a composition suitable for use as personal care fixative. This composition comprises a first polymer with anionic character - e. g. a copolymer of butyl acrylate, ethyl acrylate, methyl methacrylate, styrene and methacrylic acid - and a second polymer with cationic character - e. g. a copolymer of ethyl acrylate, methyl methacrylate, and dimethylaminoethyl methacrylate.

The problem underlying the present invention is to provide a substance with which the viscosity of an oil can be increased. Preferably, a high viscosity increase shall be achieved with a low concentration of said substance. Preferably, it shall be easy to process said substance. Preferably, a clear and easily pumpable solution of said substance in oils shall be obtainable.

This problem is solved by a composition comprising a first polymer and a second polymer, wherein the first polymer comprises repeating units derived from a monomer A which is selected from the group consisting of an alkyl ester of acrylic acid having at least 8 C-atoms, an alkyl ester of methacrylic acid having at least 9 C-atoms, and mixtures thereof, a monomer B which is an ethylenically unsaturated, polymerizable monomer having an acid group, and optionally at least one monomer C, wherein each monomer C is an ethylenically unsaturated, polymerizable monomer different from monomer A and different from monomer B, wherein the amount of monomer C in the first polymer is 0 to 20 mol-% (mol-% with respect to the mol-amount of all repeating units present in the polymer), wherein the second polymer comprises repeating units derived from a monomer D which is selected from the group consisting of an alkyl ester of acrylic acid having at least 8 C-atoms, an alkyl ester of methacrylic acid having at least 9 C-atoms, and mixtures thereof, a monomer E which is an ethylenically unsaturated, polymerizable monomer having a basic group, and optionally at least one monomer F, wherein each monomer F is an ethylenically unsaturated, polymerizable monomer different from monomer D and different from monomer E, wherein the amount of monomer F in the second polymer is 0 to 20 mol-% (mol-% with respect to the mol-amount of all repeating units present in the polymer). This composition is a subject of the present invention.

Preferred embodiments of the composition are compositions according to the dependent claims of the present text.

A further subject of the present invention is kit of parts comprising in a first container a first composition which comprises the first polymer and an oil, in a second container a second composition which comprises the second polymer and the same oil, wherein the first and second container can be combined to form a single container so that the first container is a first compartment of this single container and the second container is a second compartment of this single container.

A further subject of the present invention is the use of the composition according to the present invention or of the kit of parts according to the present invention to increase the viscosity of an oil.

The two polymers are both well soluble in a chosen hydrophobic solvent. Mixing the two components (i.e. the two polymers, each dissolved in an oil, preferably in the same oil) generates a three-dimensional network and enhances strongly the viscosity. The complementary multiple acid-base interactions between the polymeric chains is at the origin of the increase in viscosity. This technology modifies strongly the viscosity of the hydrophobic solvent even at low concentrations of the polymers used. While the interactions are reversible, the viscosified media are easily pumpable because they show shear thinning, i.e. if high shear forces are applied, the viscosity of the thickened system decreases.

A large variety of hydrophobic solvents, more or less polar can be viscosified with this system by adjusting the polymer composition. In many cases, clear or almost clear products can be obtained.

The monomer-composition of the two polymers in the composition according to the present invention provides for (1) sufficient solubility of the polymers in oils, the viscosity of which shall be increased, and (2) sufficient increase in viscosity of a composition comprising the two polymers and an oil (increase compared to the viscosity of the oil without polymers).

There are various technical areas in which increasing the viscosity of an oil is desired. One area is cosmetics, a lot of cosmetic formulations are based on oil, the viscosity of which shall be increased. Another area is the area of formulations of agrochemical active ingredients dispersed or dissolved in an oil. Another area is the area of coatings/paints and of printing inks, where achieving a suitable viscosity is important.

### Oil

The oil that can be comprised in the composition according to the present invention can by any oil. According to the present invention an oil is any substance, that is liquid at 20° C, and that is not hydrophilic. Hydrophilic in this context is a compound that is miscible with water at 20° C at any ratio and will, after mixing with water, form a single, homogenous phase together with water.

Examples of oils are mineral oil, synthetic oil, alkyl esters (the alkyl group preferably having 1 to 30 C-atoms, e. g. methyl ethylhexyl) of fatty acids (the fatty acid preferably having at least 6 C-atoms). Further examples are alkyl acetates which are not hydrophilic, e. g. cyclohexyl acetate.

In one embodiment of the present invention an oil is a substance, that is liquid at 20° C, and that has a solubility in water of not more than 1 % by weight at 20° C.

### Further cosmetic ingredients

Further cosmetic ingredients, also called cosmetically acceptable ingredients, that can be comprised in the composition according to the present invention can be one or several ingredients known to the person skilled in the art as ingredients useful in cosmetic compositions. Examples are described in the following paragraphs.

### Surfactants

Any surfactant may be present in the composition according to the present invention. In particular, a possible surfactant can be selected from the group consisting of a sulfate, an ethoxylated sulfate, a sulfonate, an alkyl polyglycoside, a derivative of an alkyl polyglycoside, a betaine, an amphoacetate, a glutamate, a sulfosuccinate, a taurate, a glycinate and an isethionate. Amongst anionic surfactants, an example is a sodium alkyl ether sulfate having 12 to 14 carbon atoms and two ethylene oxide units as ether component.

Surfactants can be anionic, non-ionic, amphoteric and/or zwitterionic. Typical examples of anionic surfactants are soaps, alkylbenzenesulfonates, alkanesulfonates, olefin-sulfonates, alkyl ether sulfonates, glycerol ether sulfonates, α-methyl ester sulfonates, sulfo fatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ethercarboxylic acids and salts thereof, fatty acid isethionates, fatty acid sarcosinates, fatty acid taurides, N-acylamino acids, for example acyl lactylates, acyl tartrates, acyl glutamates and acyl aspartates, alkyl oligoglucoside sulfates, protein fatty acid condensates (especially vegetable products based on wheat) and alkyl (ether) phosphates. If the anionic surfactants comprise polyglycol ether chains, these may have a conventional homolog distribution, but preferably have a narrow homolog distribution. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partially oxidized alk(en)yl oligoglycosides and glucuronic acid derivatives, fatty acid N-alkylglucamides, protein hydrolyzates (especially wheat-based vegetable products), polyol fatty acid esters, sugar esters, sorbitan esters, polysorbates and amine oxides. If the nonionic surfactants contain polyglycol ether chains, they may have a conventional homolog distribution, but preferably have a narrow homolog distribution. Typical examples of amphoteric or zwitterionic surfactants are alkyl betaines, alkylamido betaines, aminopropionates, aminoglycinates, imidazolinium betaines and sulfo betaines. The specified surfactants are exclusively known compounds.

### Cationic polymers

Cationic polymers may be present in the composition according to the present invention. They can be selected from the group consisting of a cationically modified cellulose derivative, PQ 10, PQ 67, a cationically modified guar derivative, guar hydroxypropyltrimonium chloride, a cationic homo- or copolymer based on acrylamide, a cationic homo- or copolymer based on vinyl pyrrolidone, a cationic homo- or copolymer based on quaternized vinyl imidazole and a cationic homo- or copolymer based on methacrylates.

Suitable cationic polymers are furthermore, for example, cationic cellulose derivatives such as a quaternized hydroxyethylcellulose, which is obtainable under the name Polymer JR 400® from Amerchol, cationic starch, copolymers of diallylammonium salts and acrylamides, quaternized vinylpyrrolidone/vinylimidazole polymers such as Luviquat® (BASF), condensation products of polyglycols and amines, quaternized collagen polypeptides such as lauryldimonium hydroxypropyl hydrolyzed collagen (Lamequat®L/Grünau), quaternized wheat polypeptides, polyethyleneimine, cationic silicon polymers such as amidomethicones, copolymers of adipic acid and dimethylaminohydroxypropyldiethylenetriamine (Cartaretine®/Sandoz), copolymers of acrylic acid with dimethyldiallylammonium chloride (Merquat® 550/Chemviron), polyaminopolyamides such as descibed in FR-A 2252840 and also crosslinked water-soluble polymers thereof, cationic chitin derivatives such as quaternized chitosan, optionally in microcrystalline distribution, condensation products of dihaloalkylene such as dibrombutane with bisdialkylamines such as bisdimethylamino-1,3-propane, cationic guar gum such as Jaguar® CBS, Jaguar® C-17, Jaguar® C-16 from Celanese, quaternized ammonium salt polymers such as Mirapol® A-15, Mirapol® AD-1, Mirapol® AZ-1 from Miranol.

### Ethoxylated fatty acid glycerides

Ehoxylated fatty acid glycerides can be present in the composition according to the present invention. They are a mixture of several individual compounds. They are obtainable by the reaction of fatty acid glycerides with ethylene oxide (EO) in alkaline conditions, in the presence of KOH for example, at elevated temperature, 100 to 150°C for example, wherein transesterifications and ethoxylations take place such that the resulting product may also comprise ethoxylated partial glycerides. In one embodiment of the present invention, ethoxylated hydrogenated castor oils are used. In one embodiment of the present invention, ethoxylated hydrogenated castor oil having 40 ethylene oxide units is used.

### Ethoxylated mono- and diglycerol esters

Ethoxylated mono- and diglycerol esters can be present in the composition according to the present invention. They are a mixture of several individual compounds. They are mixtures of molecules each comprising a glycerol residue and each comprising one or two fatty acid residues and each comprising a certain statistical average number of ethylene oxide residues.

### Waxes (also called wax bodies)

Suitable wax bodies are: alkylene glycol esters, fatty acid alkanolamides, partial glycerides, esters of polybasic, optionally hydroxy-substituted carboxylic acids, fatty alcohols, fatty ketones, fatty aledyhdes, fatty ethers, fatty carbonates, ring-opening products of olefin epoxides and mixtures thereof.

The **alkylene glycol esters** are typically mono- and/or diesters of alkylene glycols having the formula **(I)** below,

**R¹CO(OA)ₙOR²** **(I)**

in which R¹CO is a linear or branched, saturated or unsaturated acyl radical having 6 to 22 carbon atoms, R² is hydrogen or R¹CO and A is a linear or branched alkylene radical having 2 to 4 carbon atoms and n is a number from 1 to 5. Typical examples are mono- and/or diesters of ethylene glycol, propylene glycol, diethylene glycol, dipropylene glycol, triethylene glycol or tetraethylene glycol with fatty acids having 6 to 22, preferably 12 to 18 carbon atoms as: caproic acid, caprylic acid, 2-ethylhexanoic acid, capric acid, lauric acid, isotridecanoic acid, myristic acid, palmitic acid, palm oleic acid, stearic acid, isostearic acid, oleic acid, elaidic acid, petroselinic acid, linoleic acid, linolenic acid, elaeostearic acid, arachic acid, gadoleic acid, behenic acid and erucic acid and technical-grade mixtures thereof.
Other wax bodies such as **fatty acid alkanolamides**, have the formula **(II)** below,

**R³CO-NR⁴-B-OH** **(II)**

in which R³CO is a linear or branched, saturated or unsaturated acyl radical having 6 to 22 carbon atoms, R⁴ is hydrogen or an optionally hydroxy-substituted alkyl radical having 1 to 4 carbon atoms and B is a linear or branched alkylene group having 1 to 4 carbon atoms. Typical examples are condensation products of ethanolamine, methylethanolamine, diethanolamine, propanolamine, methylpropanolamine and dipropanolamine and mixtures thereof with caproic acid, caprylic acid, 2-ethylhexanoic acid, capric acid, lauric acid, isotridecanoic acid, myristic acid, palmitic acid, palm oleic acid, stearic acid, isostearic acid, oleic acid, elaidic acid, petroselinic acid, linoleic acid, linolenic acid, elaeostearic acid, arachic acid, gadoleic acid, behenic acid and erucic acid and technical-grade mixtures thereof.

**Partial glycerides** are mono and/or diesters of glycerol with linear, saturated and/or partially unsaturated fatty acids, for example, caproic acid, caprylic acid, lauric acid, myristic acid, palmitic acid, palm oleic acid, tallow fatty acid, stearic acid, behenic acid and technical-grade mixtures thereof. They are of the formula **(III)**, 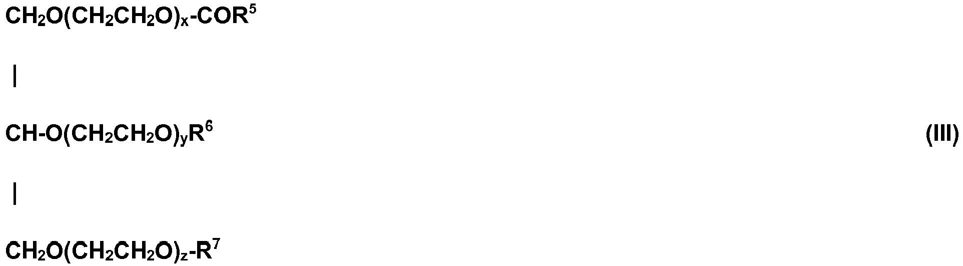 in which R⁵CO is an acyl radical having 6 to 22 carbon atoms, preferably a linear, saturated acyl radical having 6 to 22 carbon atoms, R⁶ and R⁷ are each independently hydrogen or R⁵CO, x, y and z in total are 0 or a number from 1 to 30 and X is an alkali metal or alkaline earth metal with the proviso that at least one of the two radicals R⁶ and R⁷ is hydrogen. Typical examples are lauric acid monoglyceride, lauric acid diglyceride, coconut fatty acid monoglyceride, coconut fatty acid triglyceride, palmitic acid monoglyceride, palmitic acid triglyceride, stearic acid monoglyceride, stearic acid diglyceride, tallow fatty acid monoglyceride, tallow fatty acid diglyceride, behenic acid monoglyceride, behenic acid diglyceride and technical-grade mixtures thereof which may contain minor amounts of triglyceride depending on the production process.

Also suitable as wax bodies as a preferred group are **esters of polybasic, optionally hydroxy-substituted carboxylic acids** with fatty alcohols having 6 to 22 carbon atoms. Suitable acid components of these esters are, for example, malonic acid, maleic acid, fumaric acid, adipic acid, sebacic acid, azelaic acid, dodecanedioic acid, phthalic acid, isophthalic acid and, in particular, succinic acid and also malic acid, citric acid and in particular tartaric acid and mixtures thereof. The fatty alcohols comprise 6 to 22, preferably 12 to 18 and especially 16 to 18 carbon atoms in the alkyl chain. Typical examples are caproic alcohol, capryl alcohol, 2-ethylhexyl alcohol, capric alcohol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linolyl alcohol, linolenyl alcohol, elaeostearyl alcohol, arachyl alcohol, gadoleyl alcohol, behenyl alcohol, erucyl alcohol and brassidyl alcohol and technical-grade mixtures thereof. The esters may be present as full or partial esters, preference being given to using monoesters and especially diesters of carboxylic acids or hydroxycarboxylic acids. Typical examples are succinic acid mono- and dilauryl esters, succinic acid mono- and dicetearyl esters, succinic acid mono- and distearyl esters, tartaric acid mono- and dilauryl esters, tartaric acid mono- and dicocoalkyl esters, tartaric acid mono- and dicetearyl esters, citric acid mono-, di- and trilauryl esters, citric acid mono-, di- and tricocoalkyl esters and citric acid mono-, di- and tricetearyl esters.

As a third preferred group of wax bodies, it is possible to use **fatty alcohols** having the formula **(IV)**,

**R⁸OH** **(IV)**

in which R⁸ is a linear, optionally hydroxy-substituted alkyl radical and/or acyl radical having 16 to 48, preferably 18 to 36 carbon atoms. Typical examples of suitable alcohols are cetearyl alcohol, hydroxystearyl alcohol, behenyl alcohol and oxidation products of long-chain paraffin.

**Fatty ketones**, which are suitable as components, preferably have the formula **(V)**,

**R⁹-CO-R¹⁰** **(V)**

in which R⁹ and R¹⁰ are each independently alkyl and/or alkenyl radicals having 1 to 22 carbon atoms with the proviso that they have in total at least 24 and preferably 32 to 48 carbon atoms. The ketones may be prepared by methods according to the prior art, for example by pyrolysis of the corresponding fatty acid magnesium salts. The ketones can be symmetrical or asymmetrical, preferably the two radicals R¹³ and R¹⁴ differ only by one carbon atom and are derived from fatty acids having 16 to 22 carbon atoms.

**Fatty aldehydes** suitable as wax bodies preferably correspond to the formula **(VI)**,

**R¹¹COH** **(VI)**

in which R¹¹CO is a linear or branched acyl radical having 24 to 48, preferably 28 to 32 carbon atoms.

Likewise, suitable **fatty ethers** are preferably of the formula **(VII)**,

**R¹²-O-R¹³** **(VII)**

in which R¹² and R¹³ are each independently alkyl and/or alkenyl radicals having 1 to 22 carbon atoms with the proviso that they have in total at least 24 and preferably 32 to 48 carbon atoms. Fatty ethers of the type mentioned are typically prepared by acidic condensation of the corresponding fatty alcohols. Fatty ethers with particularly advantageous pearlescent properties are obtained by condensation of fatty alcohols having 16 to 22 carbon atoms such as cetyl alcohol, cetearyl alcohol, stearyl alcohol, isostearyl alcohol, oleyl alcohol, behenyl alcohol and/or erucyl alcohol.

Suitable components are, furthermore, **fatty carbonates,** preferably of the formula **(VIII)**,

**R¹⁴O-CO-OR¹⁵** **(VIII)**

in which R¹⁴ and R¹⁵ are each independently alkyl and/or alkenyl radicals having 1 to 22 carbon atoms with the proviso that they have in total at least 24 and preferably 32 to 48 carbon atoms. The substances are obtained in a manner known per se by transesterifying, for example, dimethyl carbonate or diethyl carbonate with the corresponding fatty alcohols. Accordingly, the fatty carbonates may be symmetrical or asymmetrical. However, preference is given to using carbonates in which R¹⁴ and R¹⁵ are identical and are alkyl radicals having 16 to 22 carbon atoms. Particular preference is given to transesterification products of dimethyl carbonate or diethyl carbonate with cetyl alcohol, cetearyl alcohol, stearyl alcohol, isostearyl alcohol, oleyl alcohol, behenyl alcohol and/ or erucyl alcohol in the form of their mono- and diesters or technical-grade mixtures thereof.

**Epoxide ring-opening products** are known substances which are prepared typically by acid-catalyzed reaction of terminal or internal olefin epoxides with aliphatic alcohols. The reaction products are preferably of the formula **(IX)**, 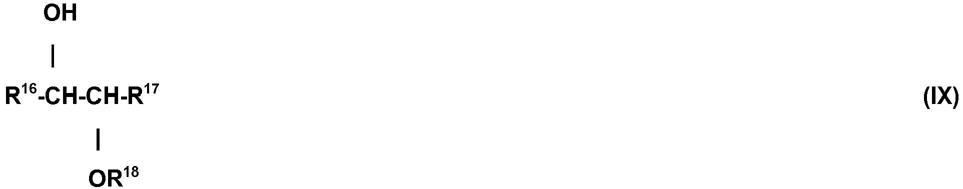 in which R¹⁶ and R¹⁷ are hydrogen or an alkyl radical having 10 to 20 carbon atoms with the proviso that the sum total of carbon atoms of R¹⁶ and R¹⁷ is in the range of 10 to 20 and R¹⁸ is an alkyl and/or alkenyl radical having 12 to 22 carbon atoms and/or is the radical of a polyol having 2 to 15 carbon atoms and 2 to 10 hydroxyl groups. Typical examples are ring-opening products of α-dodecene epoxide, α-hexadecene epoxide, α-octadecene epoxide, α-eicosene epoxide, α-docosene epoxide, i-dodecene epoxide, i-hexadecene epoxide, i-octadecene epoxide, i-eicosene epoxide and/or i-docosene epoxide with lauryl alcohol, coconut fatty alcohol, myristyl alcohol, cetyl alcohol, cetearyl alcohol, stearyl alcohol, isostearyl alcohol, oleyl alcohol, elaidyl alcohol, petroselinyl alcohol, linolyl alcohol, linolenyl alcohol, behenyl alcohol and/or erucyl alcohol. Preference is given to using ring-opening products of hexadecene and/or octadecene epoxides with fatty alcohols having 16 to 18 carbon atoms. If polyols are used for the ring opening in place of fatty alcohols, they are, for example, the following substances: glycerol; alkylene glycols such as ethylene glycol, diethylene glycol, propylene glycol, butylene glycol, hexylene glycol and polyethylene glycols having an average molecular weight of 100 to 1000 Dalton; technical-grade oligoglycerol mixtures having a degree of self-condensation of 1.5 to 10 such as technical-grade diglycerol mixtures having a diglycerol content of 40 to 50% by weight; methyol compounds such as, in particular, trimethylolethane, trimethylolpropane, trimethylolbutane, pentaerythritol and dipentaerythritol; lower alkyl glucosides particularly those having 1 to 8 carbon atoms in the alkyl radical such as methyl glucoside and butyl glucoside; sugar alcohols having 5 to 12 carbon atoms such as sorbitol or mannitol, sugars having 5 to 12 carbon atoms such as glucose or sucrose, amino sugars such as glucamine.

### Further ingredients customary in cosmetic compositions

Other useful ingredients are emulsifiers, consistency promoters, thickeners, polymers, lecithins, phospholipids, biogenic active ingredients, antidandruff agents, hydrotropes, film formers, preservatives, perfume oils and dyes.

### Examples

### Abbreviations and Definitions

- SMA: stearyl methacrylate
- EHA: 2-ethylhexyl acrylate
- MeAA: methacrylic acid
- DMAEMA: N,N-dimethylaminoethyl methacrylate
- iBoMA: isobornyl methacrylate
- Mn: number average of the molar mass
- Mw: weight average of the molar mass
- PDI: polydispersity index (means Mw/Mn)
- %: means % by weight unless defined differently
- Exxsol™ D 100: is an aromatic-free mineral oil obtainable from ExxonMobil.
- Agnique® ME 18 RD-F: is a bio-based solvent (a fatty acid methyl ester based on vegetable raw materials) obtainable from BASF.
- Cetiol® 868: is ethylhexyl stearate obtainable from BASF

The viscosity measurements were carried out at 25° C using a Brookfield viscosity LV spindle at 10 rpm, unless defined differently.

### Example 1: Polymer Example 1 (a SMA-EHA-MeAA-Copolymer)

An oil bath was heated to 75° C. In a 500 ml single-neck round-bottom flask equipped with a magnetic stir bar was added 209.7 g of methyl benzoate, 72.2 g of stearyl methacrylate (SMA), 13.4 g of 2-ethylhexyl acrylate (EHA), 4.7 g of methacrylic acid (MeAA) and 0.3 g of 2,2'-azobis(isobutyronitrile) (AIBN). The flask was sealed with a rubber septum. The mixture was gently stirred until the AIBN was fully dissolved. The monomer solution was then degassed by purging with nitrogen gas for 20 minutes. The mixture was stirred in the oil bath for ca. 15 hours to reach almost complete monomer conversion. A viscous almost colorless solution of a statistical copolymer with a solid content of 30 % was obtained (Mn = 195 kg/mol, PDI = 1.9 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 1 (2 %) in Exxsol™ D100 = 3.6 mPas
Viscosity polymer example 1 (3 %) in Exxsol™ D100 = 4.4 mPas
Viscosity polymer example 1 (4 %) in Exxsol™ D100 = 5.8 mPas
Viscosity polymer example 1 (3 %) in Agnique® ME 18 RD-F = 15 mPas
Viscosity polymer example 1 (4 %) in Agnique® ME 18 RD-F = 18 mPas

### Example 2: Polymer Example 2 (a SMA-EHA-DMAEMA-Copolymer)

An oil bath was warmed to 75° C. In a 500 mL single-neck round-bottom flask equipped with a magnetic stir bar was added 209.4 g of cyclohexyl acetate, 52.3 g of stearyl methacrylate (SMA), 29 g of 2-ethylhexyl acrylate (EHA), 9.3 g of N,N-dimethylaminoethyl methacrylate (DMAEMA) and 0.3 g of 2,2'-azobis(isobutyronitrile) (AIBN). The flask was sealed with a rubber septum. The mixture was gently stirred until AIBN was fully dissolved. The monomer solution was then degassed by purging with nitrogen gas for 10 minutes. The mixture was stirred in the oil bath for ca. 15 hours to reach almost complete monomer conversion. A slightly viscous almost colorless solution of statistical copolymer with a solid content of 30 % was obtained (Mn = 75 kg/mol, PDI = 2.7 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 2 (2 %) in Exxsol™ D100 = 4.3 mPas
Viscosity polymer example 2 (3 %) in Exxsol™ D100 = 5.2 mPas
Viscosity polymer example 2 (4 %) in Exxsol™ D100 = 6.5 mPas
Viscosity polymer example 2 (3 %) in Agnique® ME 18 RD-F = 13.5 mPas
Viscosity polymer example 2 (4 %) in Agnique® ME 18 RD-F = 16.5 mPas

### Example 3: Polymer Example 3 (a SMA-EHA-MeAA-Copolymer)

Oil bath was warmed to 75° C. In a 100 ml single-neck round-bottom flask equipped with a magnetic stir bar was added 34.9 g of methylbenzoate, 12.04 g of stearyl methacrylate (SMA), 2.23 g of 2-ethylhexyl acrylate (EHA), 0.79 g of methacrylic acid (MeAA) and 0.5 g of 2,2'-azobis(isobutyronitrile) (AIBN). The flask was sealed with a rubber septum. The mixture was gently stirred until AIBN was fully dissolved. The monomer solution was then degassed by purging with nitrogen gas for 10 minutes. The mixture was stirred in the oil bath for ca. 15 hours to reach almost complete monomer conversion. A viscous almost colorless solution of statistical copolymer with a solid content of 30 % was obtained (Mn = 88 kg/mol, PDI = 2.7 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 3 (3 %) in Exxsol™ D100 = 4.4 mPas

### Example 4: Polymer Example 4 (a SMA-DMAEMA-Copolymer)

Oil bath was warmed to 75° C. In a 100 mL single-neck round-bottom flask equipped with a magnetic stir bar was added 35 g of methyl benzoate, 6.27 g of stearyl methacrylate (SMA), 8.77 g of N,N-dimethylaminoethyl methacrylate (DMAEMA) and 0.05 g of 2,2'-azobis(isobutyronitrile) (AIBN). The flask was sealed with a rubber septum. The mixture was gently stirred until AIBN was fully dissolved. The monomer solution was then degassed by purging with nitrogen gas for 10 minutes. The mixture was stirred in the oil bath for ca. 15 hours to reach almost complete monomer conversion. A slightly viscous almost colorless solution of statistical copolymer with a solid content of 30 % was obatained (Mn = 722 kg/mol, PDI = 1.02 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 4 (3 %) in Exxsol™ D100 = 5.8 mPas
Viscosity polymer example 4 (4 %) in Exxsol™ D100 = 7.1 mPas

### Example 5: Polymer Example 5 (a SMA-EHA-MeAA-Copolymer)

Oil bath was warmed to 75° C. In a 500 ml single-neck round-bottom flask equipped with a magnetic stir bar was added 209.7 g of methyl benzoate, 72.2 g of stearyl methacrylate (SMA), 13.4 g of 2-ethylhexyl acrylate (EHA), 4.7 g of methacrylic acid (MeAA) and 0.3 g of 2,2'-azobis(isobutyronitrile) (AIBN). The flask was sealed with a rubber septum. The mixture was gently stirred until AIBN was fully dissolved. The monomer solution was then degassed by purging with nitrogen gas for 20 minutes. The mixture was stirred in the oil bath for ca. 15 hours to reach almost complete monomer conversion. A viscous almost colorless solution of statistical copolymer with a solid content of 30 % was obtained (Mn = 190 kg/mol, PDI = 1.9 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 5 (4 %) in Exxsol™ D100 = 5.9 mPas

### Example 6: Polymer Example 3 (a SMA-EHA-MeAA-Copolymer)

Oil bath was warmed to 75° C. In a 100 ml single-neck round-bottom flask equipped with a magnetic stir bar was added 34.9 g of methylbenzoate, 11.85 g of stearyl methacrylate (SMA), 2.19 g of 2-ethylhexyl acrylate (EHA), 1.02 g of methacrylic acid (MeAA), 0.1 g of n-dodecanethiol and 0.1 g of 2,2'-azobis(isobutyronitrile) (AIBN). The flask was sealed with a rubber septum. The mixture was gently stirred until AIBN was fully dissolved. The monomer solution was then degassed by purging with nitrogen gas for 10 minutes. The mixture was stirred in the oil bath for ca. 15 hours to reach almost complete monomer conversion. A viscous almost colorless solution of statistical copolymer with a solid content of 30 % was obtained (Mn = 32 kg/mol, PDI = 2.05 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 6 (4 %) in Exxsol™ D100 = 5.9 mPas

### Example 7: Polymer Example 3 (an iBoMA-EHA-MeAA-Copolymer)

In a flask was added 75.53 g of isobornyl methacrylate (iBoMA), 32.46 g of 2-ethylhexyl acrylate (EHA) und 3.93 g of methacrylic acid (MeAA). In anotherflask, 1.2 g of sodium dodecyl sulfate was dissolved in 76.7 g of water. The monomer mixture was metered to the aqueous solution and emulsified using ultra-turrax for 5 min by 20,000 rpm.

3.3 g of 5 wt.-% sodium dodecyl sulfate aqueous solution was added to 175 g of water in a polymerization vessel equipped with reflux condenser, stirrer and thermometer. The solution was warmed to ca. 78° C. Monomer emulsion and pre-charge were degassed by purging with nitrogen gas for 15 minutes. 19 g of monomer emulsion was quickly introduced in the reactor followed by 1.5 g of 5 wt.-% ammonium persulfate aqueous solution. The mixture was stirred for 20 minutes. Then, the rest of the monomer emulsion was metered in the course of 4 hours. When the addition is completed, the reaction was then subject to post-polymerization at 78° C for 60 minutes. The polymer dispersion was allowed to cool to 70° C before adding 1.65 g of 5 wt.-% Rongalit C aqueous solution and 4.13 g of 5 wt.-% of *tert-*butyl hydroperoxide. Further cooling to the room temperature was effected and the dispersion was filtered. A coagulate free product with a solid content of about 30 percent is so obtained, which was then converted to a powdered product by freeze-drying technology. (Mn = 85 kg/mol, PDI = 2.5 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 7 (3 %) in Exxsol™ D100 = 4.7 mPas

### Example 8: Polymer Example 3 (a SMA-EHA-DMAEMA-Copolymer)

Oil bath was warmed to 75° C. In a 100 mL single-neck round-bottom flask equipped with a magnetic stir bar was added 34.9 g of cyclohexyl acetate, 8.45 g of stearyl methacrylate (SMA), 4.68 g of 2-ethylhexyl acrylate (EHA), 1.97 g of N,N-dimethylaminoethyl methacrylate (DMAEMA) and 0.05 g of 2,2'-azobis(isobutyronitrile) (AIBN). The flask was sealed with a rubber septum. The mixture was gently stirred until AIBN was fully dissolved. The monomer solution was then degassed by purging with nitrogen gas for 10 minutes. The mixture was stirred in the oil bath for ca. 15 hours to reach almost complete monomer conversion. A slightly viscous almost colorless solution of statistical copolymer with a solid content of 30 % was obatained (Mn = 257 kg/mol, PDI = 1.6 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 8 (3 %) in Exxsol™ D100 = 5.8 mPas
Viscosity polymer example 8 (3 %) in Cetiol® 868 = 36 mPas
Viscosity polymer example 8 (3 %) in cyclohexyl acetate = 6 mPas

### Example 9: Polymer Example 3 (a SMA-EHA-MeAA-Copolymer)

Oil bath was warmed to 75° C. In a 250 mL single-neck round-bottom flask equipped with a magnetic stir bar was added 69.8 g of cyclohexyl acetate, 17.94 g of stearyl methacrylate (SMA), 9.98 g of 2-ethylhexyl acrylate (EHA), 2.3 g of methacrylic acid (MeAA) and 0.05 g of 2,2'-azobis(isobutyronitrile) (AIBN). The flask was sealed with a rubber septum. The mixture was gently stirred until AIBN was fully dissolved. The monomer solution was then degassed by purging with nitrogen gas for 10 minutes. The mixture was stirred in the oil bath for ca. 15 hours to reach almost complete monomer conversion. A viscous almost colorless solution of statistical copolymer was obtained with a solid content of 30 % (Mn = 65 kg/mol, PDI = 3.05 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 9 (3 %) in Cetiol® 868 = 36 mPas

### Example 10: Polymer Example 3 (a SMA-EHA-MeAA-Copolymer)

Oil bath was warmed to 75° C. In a 100 mL single-neck round-bottom flask equipped with a magnetic stir bar was added 34.9 g of cyclohexyl acetate, 8.9 g of stearyl methacrylate (SMA), 4.93 g of 2-ethylhexyl acrylate (EHA), 1.29 g of methacrylic acid (MeAA) and 0.05 g of 2,2'-azobis(isobutyronitrile) (AIBN). The flask was sealed with a rubber septum. The mixture was gently stirred until AIBN was fully dissolved. The monomer solution was then degassed by purging with nitrogen gas for 10 minutes. The mixture was stirred in the oil bath for ca. 15 hours to reach almost complete monomer conversion. A viscous almost colorless solution of statistical copolymer with a solid content of 30 % was obtained (Mn = 65 kg/mol, PDI = 3.05 determined by gel permeation chromatography using tetrahydrofuran as eluent and poly(methyl methacrylate) standards for calibration).

The viscosity of different solutions made in order to increase the viscosity of the solvent used is provided in the following list. The polymer solution obtained was diluted at a specific polymer active content written as weight percentage between brackets.
Viscosity polymer example 10 (3 %) in cyclohexyl acetate = 7 mPas

Molar compositions of example 1-10 are summarized in Table 1.

**Table 1: Molar composition of the polymers obtained in the corresponding examples**

| | **Monomer 1 (mol-%)** | **Monomer 2 (mol-%)** | **Monomer 3 (acidic monomer) (mol-%)** | **Monomer 4 (basic monomer) (mol-%)** |
|---|---|---|---|---|
| **Example 1** | SMA (63 %) | EHA (21 %) | MeAA (16 %) | - |
| **Example 2** | SMA (42 %) | EHA (42 %) | - | DMAEMA (16 %) |
| **Example 3** | SMA (63 %) | EHA (21 %) | MeAA (16 %) | - |
| **Example 4** | SMA (25 %) | - | - | DMAEMA (75 %) |
| **Example 5** | SMA (63 %) | EHA (21 %) | MeAA (16 %) | - |
| **Example 6** | SMA (60 %) | EHA (20 %) | MeAA (20 %) | - |
| **Example 7** | iBoMA (61 %) | EHA (31 %) | MeAA (8 %) | - |
| **Example 8** | SMA (40 %) | EHA (40 %) | - | DMAEMA (20 %) |
| **Example 9** | SMA (40 %) | EHA (40 %) | MeAA (20 %) | - |
| **Example 10** | SMA (39 %) | EHA (39 %) | MeAA (22 %) | - |

### Viscosity of "one-polymer-solutions" and of "two-polymers-solutions"

In examples 1 to 10 polymer powders were diluted in different solvents at a specific active concentration between 2 - 5 % as reported in these examples. Polymer solutions were diluted in respective solvents to achieve a specific active polymer concentration between 2 - 5 %. Polymer solutions with single functionality (acidic or basic) all have a low viscosity similar to the viscosity of the solvent itself.

To generate high viscosity, polymer solution B was added on polymer solution A slowly under stirring (mechanical stirrer by 400 rpm). When addition of solution B was completed, mixing was maintained for an additional minute. The viscosity was then measured either with a Brookfield viscometer DV2T with UL adapter at room temperature or with a Brookfield viscometer DV2T with LV spindle by 10rpm when the viscosity was high enough.

The description of the polymer solution mixture and the viscosity results are summarized in Table 2.

**Table 2: Viscosity of Solutions of acidic polymer plus basic polymer**

| | **Solution A and active concentration in wt.-%** | **wt.-% of Solution A in mixture** | **Solution B and active concentration in wt.-%** | **wt.-% of Solution B in mixture** | **Solvent in mixture** | **Visocity (mPas)** |
|---|---|---|---|---|---|---|
| **Mixture 1** | Example 1 (2 %) | 50 | Example 2 (2 %) | 50 | Exxsol™ D100 | 470 |
| **Mixture 2** | Example 1 (3 %) | 50 | Example 2 (3 %) | 50 | Exxsol™ D100 | 1230 |
| **Mixture 3** | Example 1 (4 %) | 50 | Example 2 (4 %) | 50 | Exxsol™ D100 | 3700 |
| **Mixture 4** | Example 2 (4 %) | 50 | Example 1 (4 %) | 50 | Exxsol™ D100 | 3600 |
| **Mixture 5** | Example 3 (3 %) | 67 | Example 4 (3 %) | 33 | Exxsol™ D100 | 6600 |
| **Mixture 6** | Example 5 (4 %) | 67 | Example 4 (4 %) | 33 | Exxsol™ D100 | 44000 |
| **Mixture 7** | Example 6 (4 %) | 50 | Example 2 (4 %) | 50 | Exxsol™ D100 | 660 |
| **Mixture 8** | Example 7 (3 %) | 50 | Example 8 (3 %) | 50 | Exxsol™ D100 | 460 |
| **Mixture 9** | Example 1 (3 %) | 50 | Example 2 (3 %) | 50 | Agnique® ME 18 RD-F | 420 |
| **Mixture 10** | Example 1 (4 %) | 50 | Example 2 (4 %) | 50 | Agnique® ME 18 RD-F | 4300 |
| **Mixture 11** | Example 9 (3 %) | 50 | Example 8 (3 %) | 50 | Cetiol® 868 | 1700 |
| **Mixture 12** | Example 10 (3 %) | 50 | Example 8 (3 %) | 50 | cyclohexyl acetate | 1550 |

The data in table 2 demonstrate that while polymer solutions with single functionality (either acidic or basic) in organic solvents have a low viscosity, the introduction of a complementary functionality drastically increase the viscosity of the system. The selection of the mixture also highlights that the viscosity can be triggered by the overall polymer concentration, as well as the specific ratio between acid and base and the molecular weight of the polymer among other parameters. This also works for a large variety of organic solvents of different polarity.

## Claims

1. A composition comprising a **first polymer** and a **second polymer,**
wherein the **first polymer** comprises repeating units derived from
a **monomer A** which is selected from the group consisting of an alkyl ester of acrylic acid having at least 8 C-atoms, an alkyl ester of methacrylic acid having at least 9 C-atoms, and mixtures thereof,
a **monomer B** which is an ethylenically unsaturated, polymerizable monomer having an acid group,
and optionally at least one **monomer C**, wherein each monomer C is an ethylenically unsaturated, polymerizable monomer different from monomer A and different from monomer B,
wherein the amount of monomer C in the first polymer is 0 to 20 mol-% (mol-% with respect to the mol-amount of all repeating units present in the polymer),
wherein the **second polymer** comprises repeating units derived from
a **monomer D** which is selected from the group consisting of an alkyl ester of acrylic acid having at least 8 C-atoms, an alkyl ester of methacrylic acid having at least 9 C-atoms, and mixtures thereof,
a **monomer E** which is an ethylenically unsaturated, polymerizable monomer having a basic group,
and optionally at least one **monomer F**, wherein each monomer F is an ethylenically unsaturated, polymerizable monomer different from monomer D and different from monomer E,
wherein the amount of monomer F in the second polymer is 0 to 20 mol-% (mol-% with respect to the mol-amount of all repeating units present in the polymer).

2. The composition according to claim 1, wherein
monomer A is selected from the group consisting of an alkyl ester of acrylic acid having 9 to 33, preferably 11 to 31, C-atoms, an alkyl ester of methacrylic acid having 10 to 34, preferably 12 to 32, C-atoms, and mixtures thereof,
monomer B is selected from the group consisting of acrylic acid, methacrylic acid and mixtures thereof,
the amount of monomer C in the first polymer is 0 to 10 mol-% (mol-% with respect to the mol-amount of all repeating units present in the polymer), preferably 0 to 5 mol-%, more preferably 0 to 2 mol-%,
monomer D is selected from the group consisting of an alkyl ester of acrylic acid having 9 to 33, preferably 11 to 31, C-atoms, an alkyl ester of methacrylic acid having 10 to 34, preferably 12 to 32, C-atoms, and mixtures thereof,
monomer E is selected from the group consisting of an alkyl acrylate having a primary, secondary or tertiary amino-group in the alkyl moiety, an alkyl methacrylate having a primary, secondary or tertiary amino-group in the alkyl moiety, and mixtures thereof, and
the amount of monomer F in the second polymer is 0 to 10 mol-% (mol-% with respect to the mol-amount of all repeating units present in the polymer), preferably 0 to 5 mol-%, more preferably 0 to 2 mol-%.

3. The composition according to claim 1, wherein
monomer A is selected from the group consisting of stearyl methacrylate, 2-ethylhexyl acrylate, isobornyl methacrylate and mixtures thereof,
monomer B is selected from the group consisting of acrylic acid, methacrylic acid and mixtures thereof,
the amount of monomer C in the first polymer is 0 to 10 mol-% (mol-% with respect to the mol-amount of all repeating units present in the polymer), preferably 0 to 5 mol-%, more preferably 0 to 2 mol-%,
monomer D is selected from the group consisting of stearyl methacrylate, 2-ethylhexyl acrylate, isobornyl methacrylate and mixtures thereof,
monomer E is N,N-dimethylaminoethyl methacrylate, and
the amount of monomer F in the second polymer is 0 to 10 mol-% (mol-% with respect to the mol-amount of all repeating units present in the polymer), preferably 0 to 5 mol-%, more preferably 0 to 2 mol-%.

4. The composition according to any of claims 1 or 3, wherein
the first monomer comprises repeating units derived from
70 to 95 mol-% monomer A (mol-% with respect to the mol-amount of repeating units derived from monomers A and B),
5 to 30 mol-% monomer B (mol-% with respect to the mol-amount of repeating units derived from monomers A and B), and
0 to 20 mol-% monomer C (mol-% with respect to the mol-amount of all repeating units present in the polymer), preferably 0 to 10 mol-%, more preferably 0 to 5 mol-%, more preferably 0 to 2 mol-%,
and wherein the second polymer comprises repeating units derived from 20 to 90 mol-% monomer D (mol-% with respect to the mol-amount of repeating units derived from monomers D and E),
10 to 80 mol-% monomer E (mol-% with respect to the mol-amount of repeating units derived from monomers D and E), and
0 to 20 mol-% monomer F (mol-% with respect to the mol-amount of all repeating units present in the polymer), preferably 0 to 10 mol-%, more preferably 0 to 5 mol-%, more preferably 0 to 2 mol-%.

5. The composition according to claim 4, wherein
the first monomer comprises repeating units derived from
78 to 92 mol-% monomer A (mol-% with respect to the mol-amount of repeating units derived from monomers A and B),
8 to 22 mol-% monomer B (mol-% with respect to the mol-amount of repeating units derived from monomers A and B), and
0 to 20 mol-% monomer C (mol-% with respect to the mol-amount of all repeating units present in the polymer), preferably 0 to 10 mol-%, more preferably 0 to 5 mol-%, more preferably 0 to 2 mol-%,
and wherein the second polymer comprises repeating units derived from 25 to 84 mol-% monomer D (mol-% with respect to the mol-amount of repeating units derived from monomers D and E),
16 to 75 mol-% monomer E (mol-% with respect to the mol-amount of repeating units derived from monomers D and E), and
0 to 20 mol-% monomer F (mol-% with respect to the mol-amount of all repeating units present in the polymer), preferably 0 to 10 mol-%, more preferably 0 to 5 mol-%, more preferably 0 to 2 mol-%.

6. The composition according to any of claims 1 to 5, wherein the number average of the molar mass of first and of the second polymer is 10 to 10000 kg/mol, preferably 30 to 800 kg/mol.

7. The composition according to any of claims 1 to 6, wherein the composition further comprises an oil, which is different from the first polymer and different from the second polymer and optionally further ingredients which are different from the first polymer, different from the second polymer, different from the oil and different from water, and optionally water.

8. The composition according to claim 7, wherein the composition comprises
0.1 to 6 % by weight of the fist polymer,
0.1 to 6 % by weight of the second polymer,
63 to 99.8 % by weight of the oil,
0 to 20 % by weight of further ingredients, preferably 0 to 10 % by weight, more preferably 0 to 5 % by weight, and
0 to 5 % by weight water, preferably 0 to 3 % by weight, more preferably 0 to 1 % by weight.

9. The composition according to claim 7, wherein the composition comprises
1 to 3 % by weight of the fist polymer,
1 to 2 % by weight of the second polymer,
73 to 98 % by weight of the oil,
0 to 20 % by weight of further ingredients, preferably 0 to 10 % by weight, more preferably 0 to 5 % by weight, and
0 to 5 % by weight water, preferably 0 to 3 % by weight, more preferably 0 to 1 % by weight.

10. The composition according to any of claims claim 7 to 9, wherein the composition is a cosmetic composition and wherein the further ingredients are cosmetic ingredients.

11. A kit of parts comprising
in a first container a first composition which comprises
the first polymer as defined in any of the previous claims and an oil,
in a second container a second composition which comprises
the second polymer as defined in any of the previous claims and the same oil, wherein the first and second container can be combined to form a single container so that the first container is a first compartment of this single container and the second container is a second compartment of this single container.

12. The use of the composition according to any of the claims 1 to 6 or of the kit of parts according to claim 11 to increase the viscosity of an oil.
